(19) Europäisches Patentamt
European Patent Office
Office européen des brevets

(11) Numéro de publication: **0 110 781**
**B1**

# (12) FASCICULE DE BREVET EUROPEEN

(45) Date de publication du fascicule du brevet: **01.04.87**

(21) Numéro de dépôt: **83402267.5**

(22) Date de dépôt: **24.11.83**

(51) Int. Cl.⁴: **C 07 D 263/58**, C 07 D 413/12, A 61 K 31/42, A 61 K 31/505

## (54) (Amino-2 éthyl)-6 benzoxazolinones substituées, leur préparation et une composition pharmaceutique les contenant.

(30) Priorité: **25.11.82 FR 8219812**

(43) Date de publication de la demande: **13.06.84 Bulletin 84/24**

(45) Mention de la délivrance du brevet: **01.04.87 Bulletin 87/14**

(84) Etats contractants désignés: **AT BE CH DE FR GB IT LI LU NL SE**

(56) Documents cités:
**EP - A - 0 049 203**
**FR - A - 2 491 471**
**US - A - 4 034 100**

(73) Titulaire: **RIOM LABORATOIRES- C.E.R.M. (Société Anonyme), Route de Marsat, F-63203 Riom Cedex (FR)**

(72) Inventeur: **Caignard, Daniel Henri, 79 B. Res. Ste Madeleine 44, Avenue J. Jaurès, F-63400 Chamalieres (FR)**
Inventeur: **Lespagnol, Charles, 115, Avenue Pasteur, F-59130 Lambersart (FR)**
Inventeur: **Lesieur, Daniel, 20, rue de Verdun, F-59147 Gondecourt (FR)**
Inventeur: **Busch, Norbert, "Le Bouquet" Loubeyrat, F-63410 Manzat (FR)**

(74) Mandataire: **Thérond, Gérard Raymond et al, RIOM LABORATOIRES - CERM - S.A. Route de Marsat B.P. 140, F-63203 Riom Cédex (FR)**

EP 0 110 781 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne de nouveaux dérivés de la benzoxazolinone substitués en 6 par une chaîne amino-2 éthyle, ainsi que leurs sels pharmaceutiquement acceptables.

Ces composés répondent à la formule générale suivante:

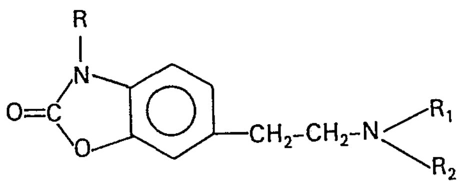

(I)

dans laquelle R représente l'hydrogène ou un radical alkyle ayant 1 à 6 atomes de carbone; $R_1$ et $R_2$ représentent séparément l'hydrogène, un radical alkyle ayant 1 à 6 atomes de carbone ou cycloalkyle ayant 3 à 7 atomes de carbone ou le radical benzyle, ou représentent ensemble avec l'atome d'azote auquel ils sont liés un radical hétérocyclique tel qu'un radical morpholino, (pyrimidino-2)-4 pipérazinyle ou phényl-4 pipérazinyle, le reste phényle pouvant lui-même être substitué par un halogène, un radical alcoxy ayant 1 à 4 atomes de carbone ou le radical trifluorométhyle.

Parmi les dérivés connus des benzoxazolinones, on peut citer la demande de brevet EP-A 49 203 concernant des composés de type I, dans lesquels le carbone de la chaîne éthylène en α du cycle benzoxazolinone porte une fonction oxo ou hydroxy. Ces composés sont revendiqués pour leurs propriétés anti-hypertensive, analgésique ou $\beta_1$ bloquante.

Par contre, les composés de formule (I) sont actifs sur le système nerveux central et possèdent plus particulièrement des propriétés hypnotiques.

De plus les composés de formule I dans lesquels $R_1$ et $R_2$ représentent tous les deux l'hydrogène peuvent être utilisés dans le traitement de l'insuffisance cardiaque. L'invention se rapporte donc aussi à l'utilisation des composés de formule I comme principe actif d'une composition pharmaceutique.

L'invention a également pour objet un procédé permettant d'obtenir lesdits composés, selon lequel on condense une amine $HNR_1R_2$ et une (halogéno-2 éthyl)-6 benzoxazolinone convenablement substituée par R conformément au schéma réactionnel suivant:

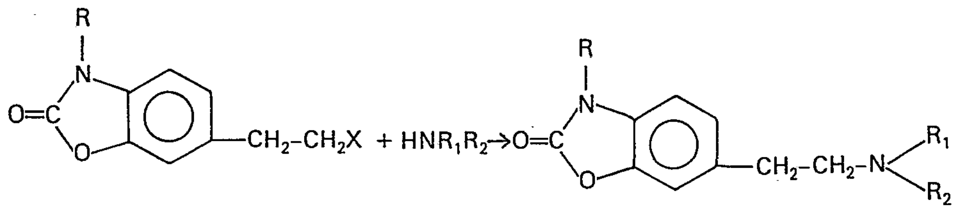


(II) (III) (I)

X représente un halogène.

La réaction se déroule au sein d'un solvant organique approprié; ce solvant est de préférence du dioxanne, mais on peut selon les cas utiliser d'autres solvants usuels, tels que le chloroforme, l'acétonitrile, le benzène ou un alcool.

Dans le cas général, la réaction s'effectue en utilisant un léger excès d'amine $NHR_2R_2$ et en chauffant le mélange réactionnel sous agitation à reflux de solvant. Après refroidissement, le précipité formé est agité dans une solution d'hydroxyde de sodium. Dans le cas où R représente l'hydrogène, le composé de l'invention se dissout et on le précipite en repassant en milieu acide, par exemple en faisant barboter un courant de dioxyde de carbone.

Dans le cas particulier où $R_1$ et $R_2$ représentent l'hydrogène, les composés correspondants sont avantageusement obtenus, soit en faisant agir sur l'(halogéno-2 éthyl)-6 benzoxazolinone l'hexaméthylène tétramine, puis en hydrolysant par chauffage en milieu acide, soit en faisant agir sur les homologues benzylés ($R_1$ = $R_2$ = $-CH_2C_6H_5$) l'hydrogène en présence de catalyseur (Pd/ charbon). Cette dernière méthode peut également avantageusement être utilisée lorsque $R_1$ = H et $R_2$ = alkyle en partant de l'homologue monobenzylé ($R_1 = -CH_2-C_6H_5$; $R_2$ = alkyle).

L'(halogéno-2 éthyl)-6 benzoxazolinone de formule II utilisée comme matière première peut elle-même être préparée à partir de l'halogénoacétyl-6 benzoxazolinone correspondante sur laquelle on fait agir le triéthylsilane en présence d'acide trifluoroacétique. Les sels pharmaceutiquement acceptables des composés de formule I sont obtenus en utilisant un acide organique ou minéral, tel que les acides chlorhydrique, hypobromique, acétique, maléique, méthanesulfonique, tartrique, fumarique, citrique.

Par «alkyle» dans la définition de R, $R_1$ et $R_2$, on entend un radical alkyle ayant 1 à 6 atomes de carbone et de préférence 1 à 4 atomes de carbone, tel que les radicaux méthyle, éthyle, isopropyle, propyle, butyle, isobutyle. Un radical cycloalkyle désigne un groupe ayant 3 à 7 atomes de carbone tels que les radicaux cyclopropyle, méthylcyclopropyle, méthylcyclobutyle et cyclohexyle.

Le radical hétérocyclique dans la définition de $R_1$ et $R_2$ est de préférence un radical hétéroaromatique à 5 ou 6 chaînons qui peut contenir un autre hétéroatome tel que l'azote ou l'oxygène.

Quand un second atome d'azote est présent

dans le radical hétérocyclique, cet atome d'azote peut être substitué. De tels radicaux hétérocycliques selon l'invention sont des radicaux pipérazinyle, (pyrimidino-2)-4-piperazinyle, ou phényl-4-pipérazinyle, dans lequel le groupe phényle peut être substitué par un halogène, un radical alcoxy ayant 1 à 4 atomes de carbone, ou le radical trifluorométhyle.

L'activité des composés de l'invention sur le système nerveux central a été recherchée chez la souris par le test d'observation permettant d'évaluer la DL 50, par le test de la motilité spontanée et sur les composés les plus représentatifs par un test confirmant les propriétés hypnotiques. Ces tests ont été mis en oeuvre conformément aux protocoles résumés ci-après.

Toxicité aiguë

Des souris de sexe mâle, de souche $OF_1$, IFFA, CREDO, d'un poids moyen de $22\pm1$ g reçoivent par intubation oesophagienne, à raison de 0,25 ml pour 20 g de poids corporel, une solution de gomme arabique et d'eau distillée (1/2 - 1/2) contenant le composé étudié. On a observé la symptomatologie de l'intoxication et relevé le mortalité au bout de 24 heures.

Motilité spontanée

Des lots de 4 souris préparées comme précédemment sont déposés dans des actimètres constitués par des couloirs circulaires coupés par des faisceaux infrarouges permettant de compter le nombre de déplacements des animaux. Le nombre de déplacements est totalisé au bout de 20 minutes et les résultats sont exprimés en pourcentage de variation par rapport au groupe d'animaux de contrôle ne recevant que le véhicule (gomme arabique - eau).

Activité hypnotique

Des souris ayant les caractéristiques précédentes reçoivent par voie intrapéritonéale 0,25 ml pour 20 g de poids corporel d'une solution constituée de gomme arabique et de soluté NaCl 0,154 (1/2 - 1/2) contenant 12,5 $mg\cdot kg^{-1}$ de composé étudié, puis on note le «temps d'endormissement» - délai d'apparition de la perte du réflexe de retournement après disposition de l'animal en décubitus dorsal et la «durée de sommeil» - durée de la perte du réflexe précédent. Pour ce test, le pentobarbital est donné comme référence, administré à la dose de 50 $mg\cdot kg^{-1}$.
Les résultats enregistrés sont rapportés dans le tableau I ci-après.

Tableau I

| Compose N°* | Estimation DL 50 $mg.kg^{-1}$ | Motilite spontanée | | Activite hypnotique | |
|---|---|---|---|---|---|
| | | Dose $mg.kg^{-1}$ | Variation % | Temps endor. en mn. | Durée sommeil en min. |
| 3 | 1000 | 50 | -97,5 | - | - |
| 4 | 1200 | 12,5 | -11,9 | - | - |
| 7 | 1200 | 50 | -69,9 | - | - |
| 8 | 1000 | 25 | -93,5 | - | - |
| 9 | 600 | 6,25 | -93,2 | 18 ± 7,5 | 46,2 ± 15 |
| 10 | 1000 | 6,25 | -99,3 | 18,2 ± 4,3 | 86 ± 15 |
| 12 | 1200 | 50 | -28 | - | - |
| 15 | 1200 | 50 | -23,2 | - | - |
| 16 | 600 | 50 | -34,2 | - | - |
| 17 | 480 | 25 | -62,8 | - | - |
| 18 | 1000 | 6,25 | -98,8 | 26,9 ± 6 | 132 ± 25 |
| 19 | 1000 | 6,25 | -97 | 15,1 ± 2 | 78 ± 16 |
| Pentobarbital | - | - | - | 9,5 ± 2,2 | 48 ± 7 |

* Voir tableau II

Ces résultats montrent que les composés de l'invention présentent une activité psychotrope, avec une facette psycholeptique, et pour certains d'entre eux (composés n°s 10, 18 et 19) une forte activité hypnotique.

Ces propriétés permettent de préconiser l'application des composés de l'invention en thérapeutique humaine pour le traitement des troubles du sommeil et des troubles du caractère et du comportement.

En plus de l'activité générale précédemment décrite, les composés de formule I dans lesquels $R_1$ et $R_2$ représentent tous les deux l'hydrogène, possèdent une activité particulièrement intéressante dans le traitement de l'insuffisance cardiaque.

Cette dernière activité a été mise en évidence en utilisant une technique derivée de celle de DEITCHMAN D. et SNYDER R.W. [Arch. Int. Pharmacodyn. 250, 65-72 (1981)].

Après anesthésie, des chiens de race commune sont placés sous respiration artificielle et appareillés de façon à enregistrer en continu la pression intraventriculaire gauche et sa dérivée dP/dt max, l'amplitude de la contraction ventriculaire gauche, le débit aortique, la fréquence cardiaque et la pression artérielle. L'insuffisance cardiaque est créée par perfusion intraveineuse

de pentobarbital à la dose de 20 mg·kg$^{-1}$min$^{-1}$ jusqu'à obtention d'une dépression de 50% de dp/dt max, puis maintenue constante en poursuivant la perfusion de pentobarbital à la dose de 0,4 mg·kg$^{-1}$min$^{-1}$. Dix minutes après l'instauration de cette insuffisance cardiaque, les produits étudiés sont administrés. Les résultats sont enregistrés comparativement à la Dopamine et la Dobutamine pris comme référence.

Dans un premier temps, on a administré les produits par voie intraveineuse: on a ainsi pu déterminer que les composés de l'invention produisaient les mêmes variations des paramètres hémodynamiques à des doses dix fois supérieures à celles des produits de référence. On a ensuite répété l'expérience en administrant les produits par voie intraduodénale en respectant le rapport d'activité de 10. La Dopamine et la Dobutamine ont été administrées à la dose de 1 mg·kg$^{-1}$ ID et les composés de l'invention à la dose de 10 mg·kg$^{-1}$. Au bout de 20 mn avec les composés de référence, on n'observe aucune variation des paramètres hémodynamiques.

Avec les composés n$^{os}$ 2 et 15, on observe au contraire une forte activité inotrope positive (augmentation de dP/dt max), une augmentation du débit aortique, ainsi qu'une récupération de la bradycardie et de l'hypotension entraînées par la perfusion de pentobarbital (action sur la fréquence cardiaque et l'hypotension).

Cette activité dans l'insuffisance cardiaque est particulièrement importante pour le composé n° 2, l'activité demeurant significative jusqu'à la soixantième minute.

Les composés de l'invention peuvent être administrés par voie enthérale ou parenthérale. En association avec les excipients pharmaceutiques habituels, les composés de formule I sont administrés à des doses journalières comprises entre 10 et 500 mg.

Le procédé d'obtention des composés de l'invention ainsi que ses différentes variantes d'exécution, est illustré plus en détails dans les exemples suivants.

Préparation de la méthyl-3 (bromo-2 éthyl)-6 benzoxazolinone

Dans une fiole à col rodé de 100 cm$^3$, on a introduit 10,8 g (0,04 mole) de méthyl-3 bromoacétyl-6 benzoxazolinone et 45,6 g (0,4 mole) d'acide trifluoroacétique puis par l'intermédiaire d'une ampoule à brome, goutte à goutte et en refroidissant, 10,5 g (0,09 mole) de triéthylsilane.

On a laissé la réaction se poursuivre pendant 15 heures sous agitation à température ambiante puis on a versé le mélange réactionnel dans 500 cm$^3$ d'eau glacée, essoré et lavé le précipité obtenu, séché et recristallisé dans le cyclohexane. On a ainsi obtenu 9,22 g de composé du titre ayant pour point de fusion F = 104,5°-105,5°C et pour analyse élémentaire:

| | C % | H % | N % |
|---|---|---|---|
| Calculé | 46,9 | 3,94 | 5,47 |
| Trouvé | 47,1 | 3,99 | 5,46 |

Exemple 1

Méthyl-3 {[(fluoro-4 phényl)-4 pipérazinyl-1]-2 éthyl}-6 benzoxazolinone

Dans une fiole rodée de 100 cm$^3$ munie d'un réfrigérant à reflux, on a introduit 0,025 mole (4,5 g) de (fluoro-4 phényl)-1 pipérazine et 0,02 mole (5,12 g) de méthyl-3 (bromo-2 éthyl)-6 benzoxazolinone préalablement dissoute dans 50 cm$^3$ de dioxanne, puis on a chaffé à reflux sous agitation pendant 72 heures. Après refroidissement, essorage et séchage, on a ajouté au précipité obtenu 100 cm$^3$ d'une solution aqueuse d'hydroxyde de sodium à 3% et maintenu sous agitation pendant 30 minutes. Après essorage, séchage et recristallisation dans le toluène, on a obtenu 4,6 g de produit du titre ayant pour point de fusion F = 161–164°C et pour analyse élémentaire:

| | C % | H % | N % |
|---|---|---|---|
| Calculé | 67,59 | 6,24 | 11,82 |
| Trouvé | 67,87 | 6,18 | 11,73 |

Exemple 2

Méthyl-3 {[(méthoxy-2 phényl)-4 pipérazinyl] -2 éthyl} -6 benzoxazolinone

En procédant comme indiqué dans l'exemple 1, mais en partant de 5,12 g de méthyl-3 (bromo-2 éthyl)-6 benzoxazolinone et de 4,82 g de (méthoxy-2 phényl)-1 pipérazine, on a obtenu après recristallisation dans le cyclohexane 4,78 g de produit du titre ayant pour point de fusion F = 137–137,5°C et pour analyse élémentaire:

| | C % | H % | N % |
|---|---|---|---|
| Calculé | 68,54 | 6,86 | 11,44 |
| Trouvé | 68,50 | 6,70 | 11,56 |

Exemple 3

{[(Méthoxy-2 phényl)-4 pipérazinyl-1]2-éthyl} -6 benzoxazolinone

Dans une fiole à col rodé de 100 cm$^3$ munie d'un réfrigérant à reflux, on a introduit 0,025 mole (4,82 g) de (méthoxy-2 phényl)-1 pipérazine et 0,02 mole (4,85 g) de (bromo-2 éthyl)-6 benzoxazolinone préalablement dissoute dans 50 cm$^3$ de dioxanne, puis on a chauffé à reflux sous agitation pendant 72 heures. Après refroidissement, essorage et séchage, on a dissous le précipité formé dans la quantité suffisante d'une solution aqueuse d'hydroxyde de sodium à 3%. On a ensuite fait barboter un courant de dioxyde de carbone, essoré, lavé et séché le précipité. Après recristallisation dans le toluène, on a obtenu 4,7 g de produit du titre ayant pour point de fusion F = 182–184°C et pour analyse élémentaire:

| | C % | H % | N % |
|---|---|---|---|
| Calculé | 67,97 | 6,56 | 11,89 |
| Trouvé | 67,89 | 6,67 | 11,68 |

Exemple 4

{[(pyrimidino-2)-4 pipérazinyl-1]-2 éthyl} -6 benzoxazolinone

En procédant comme indiqué dans l'exemple 3, mais en partant de 4,10 g de (pyrimidino-2)-1 pipérazine et de 4,85 g de (bromo-2 éthyl)-6 benzoxazolinone, on a obtenu 4,2 g de produit du titre ayant pour point de fusion F = 189–189,5°C et pour analyse élémentaire:

| | C% | H% | N% |
|---|---|---|---|
| Calculé | 62,75 | 5,89 | 21,53 |
| Trouvé | 62,36 | 5,88 | 21,27 |

Exemple 5

Méthyl-3 (amino-2 éthyl)-6 benzoxazolinone (Chlorhydrate)

Dans une fiole à col rodé de 250 $cm^3$ munie d'un réfrigérant à reflux, on a introduit 0,04 mole (5,60 g) d'héxaméthylène tétramine préalablement dissous dans 55 $cm^3$ de chloroforme et 0,03 mole (7,7 g) de méthyl-3 (bromo-2 éthyl)-6 benzoxazolinone préalablement dissous dans 30 $cm^3$ de chloroforme, puis on a chauffé à reflux sous agitation pendant 150 heures. Après essorage et séchage, on a introduit le précipité dans une fiole à col rodé munie d'un réfrigérant à reflux, et on a ajouté 150 $cm^3$ d'alcool absolu et 30 $cm^3$ d'acide chlorhydrique concentré. Après avoir chauffé à reflux pendant deux heures, on a éliminé le solvant, lavé le résidu à l'alcool absolu, séché et recristallisé dans le méthanol.

On a ainsi obtenu 4,46 g du composé du titre sous forme de chlorhydrate ayant pour point de fusion F = 243°C (décomposition) et pour analyse élémentaire:

| | C% | H% | N% |
|---|---|---|---|
| Calculé | 52,52 | 5,73 | 12,25 |
| Trouvé | 52,15 | 5,70 | 12,27 |

Example 6

Méthyl-3 (isopropylamino-2 éthyl)-6 benzoxazolinone (Bromhydrate)

Dans une fiole à col rodé de 100 $cm^3$ munie d'un réfrigérant à reflux, on a introduit 0,2 mole (11,82 g) d'isopropylamine et 0,02 mole (5,12 g) de méthyl-3 (bromo-2 éthyl)-6 benzoxazolinone préalablement dissous dans 60 $cm^3$ d'acétonitrile, puis on a chauffé à reflux pendant 15 heures sous agitation.

Après refroidissement, essorage, séchage et recristallisation dans l'alcool à 95°, on a obtenu 5,36 g de composé du titre sous forme de bromhydrate ayant pour point de fusion F = 265°C (décomposition) et pour analyse élémentaire:

| | C% | H% | N% |
|---|---|---|---|
| Calculé | 49,53 | 6,08 | 8,89 |
| Trouvé | 49,25 | 6,01 | 8,81 |

Exemple 7

Méthyl-3 [(N-benzyl N-méthyl amino)-2 éthyl]-6 benzoxazolinone

En procédant comme indiqué dans l'exemple 6, mais en partant de 0,04 mole (4,90 g) de N-benzyl N-méthyl amine et de 0,02 mole (5,10 g) de méthyl-3 (bromo-2 éthyl)-6 benzoxazolinone, on a obtenu après recristallisation dans l'hexane 3,85 g de composé du titre ayant pour point de fusion F = 72–73°C et pour analyse élémentaire:

| | C% | H% | N% |
|---|---|---|---|
| Calculé | 72,95 | 6,80 | 9,45 |
| Trouvé | 72,90 | 6,82 | 9,34 |

Exemple 8

Méthyl-3 (méthylamino-2 éthyl)-6 benzoxazolinone (Chlorhydrate)

Dans une fiole à col rodé de 1000 $cm^3$, on a dissous 0,025 mole (7,4 g) du composé préparé à l'exemple 7 dans 400 $cm^3$ de méthanol, puis on a introduit 0,35 g de charbon palladié et agité sous atmosphère d'hydrogène à température et pression ordinaire. Après absorption de la quantité théorique d'hydrogène (560 $cm^3$ en dix heures), on a filtré le milieu réactionnel, concentré le filtrat et acidifié par l'acide chlorhydrique gazeux.

Après essorage du précipité et recristallisation dans le méthanol, on a obtenu 4,55 g de composé du titre sous forme de chlorhydrate dont le point de fusion était supérieur à 265°C (décomposition) et ayant pour analyse élémentaire:

| | C% | H% | N% |
|---|---|---|---|
| Calculé | 54,43 | 6,23 | 11,54 |
| Trouvé | 54,51 | 6,22 | 11,50 |

Exemple 9

Méthyl-3 (dipropylamino-2 éthyl)-6 benzoxazolinone

Dans une fiole à col rodé de 100 $cm^3$ munie d'un réfrigérant à reflux, on a introduit 2,02 g (0,02 mole) de n, n dipropylamine et 2,56 g (0,01 mole) de méthyl-3 (bromo-2 éthyl)-6 benzoxazolinone préalablement dissous dans 30 $cm^3$ de dioxanne, puis on a chaffé à reflux sous agitation pendant soixante douze heures. Après refroidissement, on a essoré le mélange réactionnel, puis évaporé le filtrat au bain-marie sous vide. On a ensuite repris le résidu par 30 $cm^3$ d'éther diéthylique, filtré et lavé le filtrat à l'eau, séché sur chlorure de calcium, puis fait barboter un courant d'acide chlorhydrique gazeux. Le précipité formé a été essoré, puis recristallisé dans l'acétate d'éthyle et on a obtenu sous forme de chlorhydrate 2,23 g de composé du titre ayant pour point de fusion F = 150–152°C et pour analyse élémentaire:

| | C% | H% | N% |
|---|---|---|---|
| Calculé | 60,13 | 8,12 | 8,77 |
| Trouvé | 60,15 | 8,18 | 8,71 |

Selon les mêmes procédés, on a également préparé les composés dont les caractéristiques sont indiquées dans le tableau II ci-après.

Tableau II

| Compose N° | R | 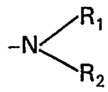 | SEL | F°C |
|---|---|---|---|---|
| 1 (Exemple 6) | $-CH_3$ | 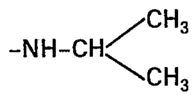 | Bromhydrate | 265 (décomposition) |
| 2 (Exemple 5) | $-CH_3$ | $-NH_2$ | Chlorhydrate | 243 (décomposition) |
| 3 | $-CH_3$ | 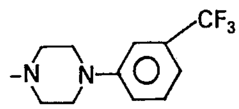 | Base | 108,5–109,5 |
| 4 | $-CH_3$ | 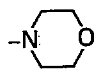 | Chlorhydrate | 265 |
| 5 (Exemple 7) | $-CH_3$ | 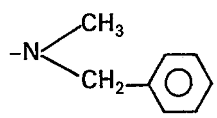 | Base | 72–73 |
| 6 (Exemple 8) | $-CH_3$ | $-NH-CH_3$ | Chlorhydrate | > 265 (décomposition) |
| 7 | $-CH_3$ | 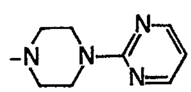 | Base | 170,5–171,5 |
| 8 | -H | 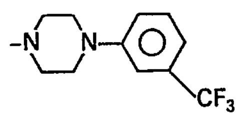 | Base | 146–148 |
| 9 (Exemple 2) | $-CH_3$ | 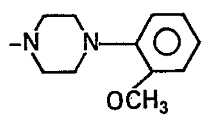 | Base | 137–137,5 |
| 10 (Exemple 3) | -H | 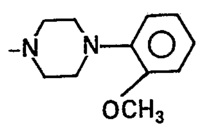 | Base | 182–184 |
| 11 | -H | 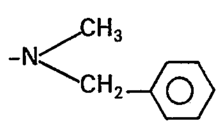 | Hémihydrate | 131–133 |
| 12 | -H | $-NH-CH_3$ | Chlorhydrate | 265 (décomposition) |
| 13 | -H | 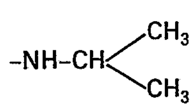 | Bromhydrate | 264 |

Tableau II (Suite)

| Compose N° | R | 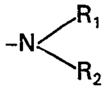 | SEL | F°C |
|---|---|---|---|---|
| 14 | -H | 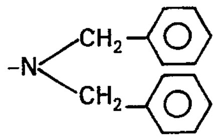 | Base | 181–183 |
| 15 | -H | $-NH_2$ | Chlorhydrate | 265 (décomposition) |
| 16 | $-CH_3$ | 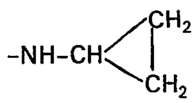 | Bromhydrate | 250 (décomposition) |
| 17 (Exemple 4) | -H | 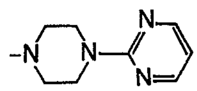 | Base | 189–189,5 |
| 18 (Exemple 1) | $-CH_3$ | 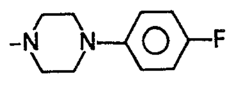 | Base | 161–164 |
| 19 | -H | 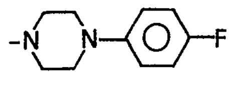 | Base | 201-203 |
| 20 | $-CH_3$ | 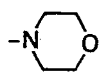 | Base | 156–159 |
| 21 (Exemple 9) | $-CH_3$ | 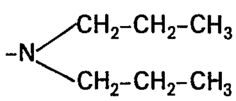 | Chlorhydrate ⅜ $H_2O$ | 150–152 |

**Revendications**

1. Composés caractérisés en ce qu'ils répondent à la formule:

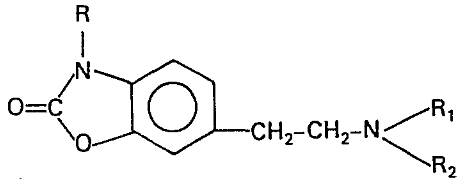

(I)

dans laquelle R représente l'hydrogène ou un radical alkyle ayant 1 à 6 atomes de carbone, $R_1$ et $R_2$ représentent séparément l'hydrogène, un radical alkyle ayant 1 à 6 atomes de carbone ou cycloalkyle ayant 3 à 7 atomes de carbone ou le radical benzyle ou représentent ensemble avec l'atome d'azote auquel ils sont liés un radical hétérocyclique, tel que les radicaux morpholino, (pyrimidino-2)-4 pipérazinyle ou phényl-4 pipérazinyle, le reste phényle pouvant lui-même être substitué par un halogène, un radical alcoxy ayant 1 à 4 atomes de carbone ou le radical trifluorométhyle, et leurs sels pharmaceutiquement acceptables.

2. Composés selon la revendication 1 caractérisés en ce que la définition alkyle pour les radicaux R, $R_1$, $R_2$ correspond à un radical ayant 1 à 4 atomes de carbone.

3. Composés selon la revendication 1, caractérisés en ce que $R_1$ et $R_2$ représentent avec l'atome

d'azote auquel ils sont liés l'un des radicaux (méthoxy-2 phényl) ou (trifluoro-3 phényl) ou (fluoro-4 phényl)-4 pipérazinyle, et R représente soit l'hydrogène, soit le radical méthyle.

4. Composés selon la revendication 1, caractérisés en ce que $R_1$ représente l'hydrogène et $R_2$ représente l'un des radicaux méthyle, isopropyle ou cyclopropyle.

5. Composés selon la revendication 1 caractérisés en ce que $R_1$ et $R_2$ représentent chacun l'hydrogène.

6. Procédé d'obtention des composés selon l'une quelconque des revendications 1 à 5 caractérisé en ce qu'on condense une amine $NHR_1R_2$ et une (halogéno-2 éthyl)-6 benzoxazolinone.

7. Procédé selon la revendication 6 caractérisé en ce que le dérivé halogéné utilisé est une (bromo-2 éthyl)-6 benzoxazolinone.

8. Procédé selon la revendication 6 pour la préparation d'un composé selon la revendication 5 caractérisé en ce qu'on fait agir l'hexaméthylène tétramine avec une (halogéno-2 éthyl)-6 benzoxazolinone.

9. Procédé selon la revendication 6 pour la préparation d'un composé selon la revendication 5 caractérisé en ce qu'on fait réagir une dibenzylamine avec une (halogéno-2 éthyl)-6 benzoxazolinone, puis on soumet le composé obtenu à une réduction.

10. Composition pharmaceutique utile en tant qu'hypnotique caractérisée en ce qu'elle comprend à titre de principe actif au moins un composé selon l'une quelconque des revendications 1 à 5 en association avec des excipients appropriés.

11. Composition pharmaceutique utile dans le traitement de l'insuffisance cardiaque caractérisée en ce qu'elle comprend à titre de principe actif un composé selon la revendication 5 en association avec des excipients appropriés.

**Patentansprüche**

1. Verbindungen, dadurch gekennzeichnet, dass sie der Formel

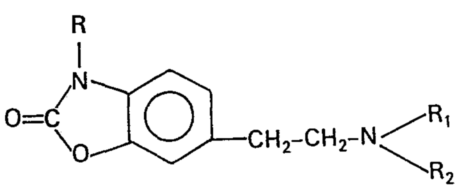

(I)

entsprechen, worin R für Wasserstoff oder einen Alkylrest mit 1 bis 6 Kohlenstoffatomen, $R_1$ und $R_2$ unabhängig voneinander für Wasserstoff, einen Alkylrest mit 1 bis 6 Kohlenstoffatomen oder Cycloalkyl mit 3 bis 7 Kohlenstoffatomen oder den Benzylrest oder zusammen mit dem Stickstoffatom, an das sie gebunden sind, für einen heterocyclischen Rest, wie einen Morpholino-, 4-Pyrimidino-(2)-pi perazinyl- oder 4-Phenylpiperazinylrest stehen, wobei der Phenylrest seinerseits durch ein Halogen, einen Alkoxyrest mit 1 bis 4 Kohlenstoffatomen oder den Trifluormethylrest substituiert sein kann, und deren pharmazeutisch unbedenkliche Salze.

2. Verbindungen nach Anspruch 1, dadurch gekennzeichnet, dass die Definition der Reste R, $R_1$ und $R_2$ als Alkyl einem Rest mit 1 bis 4 Kohlenstoffatomen entspricht.

3. Verbindungen nach Anspruch 1, dadurch gekennzeichnet, dass $R_1$ und $R_2$ zusammen mit dem Stickstoffatom, an das sie gebunden sind, für einen der Reste 4-(2-Methoxy-phenyl)-, 4-(3-Trifluormethylphenyl)- oder 4-(4-Fluorphenyl)-piperazinyl stehen und R entweder Wasserstoff oder den Methylrest darstellt.

4. Verbindungen nach Anspruch 1, dadurch gekennzeichnet, dass $R_1$ für Wasserstoff und $R_2$ für einen der Reste Methyl, Isopropyl oder Cyclopropyl stehen.

5. Verbindungen nach Anspruch 1, dadurch gekennzeichnet, dass $R_1$ und $R_2$ je für Wasserstoff stehen.

6. Verfahren zur Herstellung der Verbindungen nach einem der Ansprüche 1 bis 5, dadurch gekennzeichnet, dass man ein Amin $NHR_1R_2$ mit einem 6-(2-Halogenäthyl)-benzoxazolinon kondensiert.

7. Verfahren nach Anspruch 6, dadurch gekennzeichnet, dass man als halogeniertes Derivat 6-(2-Bromäthyl)-benzoxazolinon einsetzt.

8. Verfahren nach Anspruch 6 zur Herstellung einer Verbindung nach Anspruch 5, dadurch gekennzeichnet, dass man Hexamethylentetramin mit einem 6-(2-Halogenäthyl)-benzoxazolinon reagieren lässt.

9. Verfahren nach Anspruch 6 zur Herstellung einer Verbindung nach Anspruch 5, dadurch gekennzeichnet, dass man Dibenzylamin mit einem 6-(2-Halogenäthyl)-benzoxazolinon zur Reaktion bringt und die erhaltene Verbindung dann einer Reduktion unterzieht.

10. Als Schlafmittel verwendbare pharmazeutische Zusammensetzung, dadurch gekennzeichnet, dass sie als Wirkstoff mindestens eine Verbindung nach einem der Ansprüche 1 bis 5 zusammen mit geeigneten Trägern enthält.

11. Zur Behandlung von Herzinsuffizienz verwendbare pharmazeutische Zusammensetzung, dadurch gekennzeichnet, dass sie als Wirkstoff eine Verbindung nach Anspruch 5 zusammen mit geeigneten Trägern enthält.

**Claims**

1. Compounds of the formula

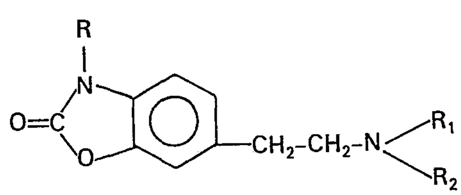

(I)

in which R represents hydrogen or an alkyl radical with 1 to 6 carbon atoms, $R_1$ and $R_2$ each re-

present hydrogen, an alkyl radical with 1 to 6 carbon atoms or cycloalkyl with 3 to 7 carbon atoms or the benzyl radical or $R_1$ and $R_2$ together with the nitrogen atom to which they are connected represent a heterocyclic radical such as morpholino, 4-(2-pyrimidino)-piperazinyl, or 4-phenylpiperazinyl, whereby the phenyl radical itself may be substituted by halogen, an alkoxy radical with 1 to 4 carbon atoms or the trifluoromethyl radical, as well as the pharmaceutically acceptable salts thereof.

2. Compounds according to claim 1 characterised in that the alkyl meaning in the definitions of R, $R_1$ and $R_2$ represents an alkyl radical with 1 to 4 carbon atoms.

3. Compounds according to claim 1 characterised in that $R_1$ and $R_2$ together with the nitrogen atom to which they are connected, represent a radical selected from 4-(2-methoxy phenyl)-piperazinyl, 4-(3-trifluoromethylphenyl)-piperazinyl and 4-(4-fluorophenyl)-piperazinyl, and R represents either hydrogen or the methyl radical.

4. Compounds according to claim 1 characterised in that $R_1$ represents hydrogen an $R_2$ methyl, isopropyl or cyclopropyl.

5. Compounds according to claim 1 characterised in that $R_1$ and $R_2$ each represent hydrogen.

6. Process for the preparation of the compounds according to any of the claims 1 to 5 characterised in that an amine of the formula $NHR_1R_2$ is condensed with a 6-(2-halogen ethyl)-benzoxazolinone.

7. Process according to claim 6 characterised in that as the halogenated derivative is used 6-(2-bromoethyl)-benzoxazolinone.

8. Process according to claim 6 for the preparation of a compound according to claim 5 characterised in that hexamethylentetramine is reacted with a 6-(2-halogen ethyl)-benzoxazolinone.

9. Process according to claim 6 for the preparation of a compound according to claim 5, characterised in that a dibenzylamine is reacted with a 6-(2-halogen ethyl)-benzoxazolinone after which the compound thus obtained is reduced.

10. Pharmaceutical composition useful as hypnotic characterised in that it contains at least a compound according to any of the claims 1 to 5 as the acitve principle together with suitable carriers.

11. Pharmaceutical composition useful in treating cardiac insufficiencies characterised in that it contains a compound according to claim 5 as the active principle together with suitable carriers.